Europäisches Patentamt

(19) European Patent Office  (11) Publication number: **0 031 318**

Office européen des brevets  **A1**

(12) **EUROPEAN PATENT APPLICATION**

(21) Application number: 81100251.8  (51) Int. Cl.³: **A 24 C 1/04**

(22) Date of filing: 05.03.79  **B 26 D 5/34**

(30) Priority: 09.03.78 US 884849
02.05.78 US 902262

(43) Date of publication of application:
01.07.81 Bulletin 81/26

(84) Designated Contracting States:
BE CH DE FR GB IT LU NL SE

(60) Publication number of the earlier application
in accordance with Art. 76 EPC:: 0 004 170

(71) Applicant: GULF & WESTERN CORPORATION
1 Gulf & Western Plaza
New York, N.Y. 10023(US)

(72) Inventor: Anderson, Verner
R.D. No.3-3103
Berwick Pennsylvania 18603(US)

(72) Inventor: Logan, David J.
125 Karen Lee Road
Glastonbury Connecticut 06133(US)

(72) Inventor: Wood, Kenneth O.
27 Ludwig Road
Ellington Tolland Connecticut 06029(US)

(74) Representative: Abbie, Andrew Kenneth
A.A. THORNTON & CO. Northumberland House 303/306
High Holborn
London, WC1V 7LE(GB)

(54) Apparatus for cutting an element from a natural tobacco leaf.

㉗ An apparatus for cutting cigar wrappers with a preselected shape from a natural tobacco leaf (L) comprises a vacuum supporting structure on a leaf receiving member (300). An arrangement is provided for selecting a cut position on the leaf and oriented with respect to the leaf receiving member (300) and a cutting table (750) is provided including a leaf supporting surface (752b) and an arrangement for creating a vacuum on the surface to support a leaf. The table is positioned in a known leaf receiving location and a transfer means (202) is provided for transferring the leaf from the leaf receiving member (300) to the leaf supporting surface of the cutting table. A cutter (810a) is provided for cutting the preselected shape from the leaf. The table (750) is then shifted from its leaf receiving position to a leaf cutting position with the support surface (752b) of the table (750) spaced from the wrapper cutter (810a). After orienting the platen relative to the cutter to align the cutter with the cut position for the leaf the cutter is forced against the table (750) to cut a wrapper from the leaf. The wrapper is then moved and transferred by transfer device (870) to a selected location for subsequent use.

EP 0 031 318 A1

./...

FIG. 7

# - 1 -
## Apparatus for cutting an element from a
## natural tobacco leaf

Attention is directed to the claims of copending Application No. 79300323.7 from which this application is divided and also to the claims of Applications Nos.            ,                  and which are also divided therefrom.

The present invention relates to cutting elements, such as cigar wrappers, having preselected shapes from a natural tobacco leaf.

In the production of cigars, a core is usually provided. In practice, the core is formed either of small tobacco pieces or whole tobacco leaves bunched in a longitudinal direction. These cores are usually wrapped with a binder and then spirally wrapped by an elongated sheet, known as a "wrapper". The cigar wrapper is formed by cutting a given profile from a tobacco sheet product. In some instances, a wrapper is cut from a synthetic tobacco sheet. However, it is desirable to provide a natural tobacco leaf as the outer cigar wrapper to present an appealing appearance which facilitates marketing of the finished cigar. It is essential for a quality cigar that a natural tobacco leaf be used for the outer wrapper and that the finished cigar have no noticeable defect, such as a hole, a colour variation, an edge portion of the leaf, or a stem. Indeed, certain unduly noticeable heavy veins should not be incorporated into a wrapper for a quality cigar. All of these concepts in wrapper selection are essential in the marketing of a cigar in the very competitive cigar industry. The wrapper is important in determining the overall visual concept that a purchaser forms regarding the quality of the cigar. Consequently, the wrapper must have an appearance that imparts an impression of quality to the resulting cigar.

As is well known in the cigar industry, the wrapper, which is helically disposed in overlapping fashion around the cigar, must also have a smooth outer appearance which is somewhat difficult to obtain since the shape of the cigar often varies along its length. For that reason, the wrapper must be cut in a complex shape and must be accurately spiralled around the cigar core and/or binder to produce the desired smooth outer appearance. This requirement, combined with the demand for a cigar wrapper with no surface defects, has made one of the more critical aspects of cigar making the system by which the cigar wrapper is cut from a natural tobacco leaf. This system is made quite complex by the fact that each natural tobacco leaf has different surface variations which must be excluded from a wrapper.

Due to the extreme criticality in producing quality cigar wrappers for use in cigar manufacturing, the wrappers are generally cut by a manual orienting procedure that has not changed substantially over the years. The process requires a skilled operator who manually orients a half of a leaf formed by removing the centre stem. This leaf portion or half is examined for holes, coarse veins, or other visible imperfections on one surface. After this inspection, the leaf portion is usually spread onto a cutting surface including a cutting die surrounded by perforated surfaces through which vacuum can be applied to the spread leaf. The leaf is manually positioned over the cutting die to ensure that the outline of the die, which has the shape of the cigar wrapper, does not include an edge portion or any other visible surface imperfection in the natural tobacco leaf. After this placement has been made, the vacuum is applied to hold the leaf in place on the cutting surface. A roller is forced over the leaf and cuts out a leaf portion determined by the profile of the cutter over which the leaf was positioned. After a first cut has been made, the vacuum is released, the wrapper is removed and the cut tobacco portion is again oriented by the operator for a second cut from the leaf half, if a second cut is possible without including any surface imperfection. The wrapper has an elongated shape and the leaf veins must have a predetermined diagonal orientation in the wrapper. Consequently, the general disposition of the wrapper cut must be generally parallel to the original stem of the natural tobacco leaf within a few degrees, such as $10^{o}-15^{o}$. In this manner, the veins, which are found on the leaf, will have the proper pattern when the wrapper is spirally wound around the core and/or binder to form a finished cigar. This process of manually orienting and then cutting is continued until no other wrapper can be cut from a leaf half. At this time, the leaf is removed from the cutting station for use in other tobacco products or discarded. Other procedures are used in the tobacco industry for producing the cigar wrappers. This particular description is representative in that each of the procedures involves manual manipulation of a leaf or a leaf half into a particular position wherein a cut is made in the natural tobacco leaf so as to avoid surface imperfections. In all instances, an operator, who must be skilled and highly trained, is required for the production of a quality wrapper produced from a natural tobacco leaf. In practice, the cost of producing the quality wrapper is a relatively high proportion of the cigar manufacturing costs in that the remainder of the cigar making process is generally mechanized and can be accomplished at relatively high processing speeds.

In view of this, there is a substantial demand for an arrangement wherein the cigar wrapper can be cut from a tobacco leaf in a high speed operation involving no manual manipulation without sacrificing the high quality required for the production of such wrappers. The advantage to the cigar manufacturing process of avoiding, or reducing, the manual manipulation required in producing the cigar wrappers is well known in the industry.

Before the leaves are stacked for use by an operator, the heavy stem or mid-rib of each tobacco leaf is removed. Each resulting half of the leaf is then "booked" in a separate pile for use in the cutting operation. Since the veins in the leaf extend diagonally in different general directions with respect to the stem, one half of the leaf is used for one cigar making machine and the other half of the leaf is used for another cigar making machine. This prevents mixing of wrappers from both leaf halves so that the diagonal veins within the cigar wrapper are uniform for each run of cigars. If whole leaves were provided to the operator for manual orientation and cutting, the cut wrappers could have different vein patterns unless the operator exercised extreme care and attention. Such mixing of vein patterns would not be acceptable in the production of cigars. The wrappers must be consistent in the orientation of the vein angles. Consequently, not only have prior arrangements for cutting cigar wrappers required manual manipulation, but they have required stemming of the tobacco leaf and grouping the leaves in "booked" and matched halves for use in a particular tobacco wrapping machine. Stemming, booking and other controls have increased the cost of wrappers without increasing their quality. All of these disadvantages are known in the cigar industry and attempts have been made to correct one or more of the various disadvantages experienced in the previously used arrangements for producing cigar wrappers from natural tobacco leaves.

The most common approach to solving the problems in cutting wrappers has been to mechanize or increase the speed of the cutting operation and the wrapper transfer operation. Such a concept is shown in United States Letters Patent 3,591,044. This patent is incorporated herein by reference for background information only. Such an arrangement increases production by an operator, but it does not solve the basic problems involved in the efficient production of a wrapper from a natural tobacco leaf. Manual manipulation or orientation, stemming and booking of leaf halves are still required. This was the background situation presented when the present novel system was developed to cut cigar wrappers from natural tobacco leaves and the preferred embodiment of the present invention comprises an apparatus for cutting cigar wrappers from natural tobacco leaves especially in a system wherein the cutting position is selected according to defects in the leaf. However, the invention has broader applications.

In accordance with the invention, there is provided an apparatus for cutting an element having a preselected shape from a natural tobacco leaf, said apparatus comprising: vacuum means for supporting said leaf in a spread condition and in a random position on a leaf receiving member; means for locating at least one cut position on said leaf and oriented with respect to said leaf receiving member; a cutting table including a leaf support surface and means for creating a vacuum at said leaf support surface; means for positioning said table in a known leaf receiving position with respect to said leaf receiving member; transfer means for transferring said leaf from said leaf receiving member to said leaf support surface when said cutting table is in said leaf receiving position; a cutter for cutting said preselected shape; means for shifting said table from said leaf receiving position to a leaf cutting position whereat said support surface of said table is spaced from said cutter; means for orienting said table relative to said cutter to a position with said cutter aligned with said selected cut position; means for forcing said cutter against said table whereby an element is cut and separated from said leaf; and means for then transferring said cut and separated element to a selected location for subsequent use.

In the preferred apparatus, the cutter forcing means is arranged to force a member carrying a plurality of said cutters, each with a cutting shape corresponding to one of a plurality of preselected shapes, toward said leaf support surface and respective means for selectively shifting each of said cutters with respect to said member between a retracted non-cutting position and an extended cutting position.

Each of the shifting means in the preferred apparatus comprises a cam coacting with one of said cutters and said member carrying said cutters and means for shifting said cam between a first position with said cutter adjacent said member and a second position with said cutter extended from said member.

The present invention thus enables a natural leaf to be oriented with respect to one surface and then transferred to another surface which is in a fixed, known position. Thereafter, the other member which is the cutting table can be shifted to the cutting position and a cut can be made. By orienting the two members prior to the transfer, the leaf is on the cutting table in a preselected position which allows easy shifting of the cutting table to the desired cutting position. Also, by providing a number of cutters each having a preselected cutting shape on a single member, as in the preferred embodiment, the cutter being used for a particular cut can be selected conveniently by extending the desired cutter while the remaining cutters are in a retracted position. This causes the cutter that is extended to perform the cutting operation, while the remaining cutters remain in their inactive positions. This provides a convenient manner for selecting a desired cutter having a given profile for each cut of an apparatus of the type described above.

In order that the invention may be well understood, an embodiment thereof, which is given by way of example only, will now be described with reference to the accompanying drawings, in which:

Figure 1 is a top plan view illustrating a natural tobacco leaf from which the wrappers are to be cut;

Figure 2 is a schematic view illustrating the shape of the cigar wrapper to be cut together with the encompassing profile or facsimile shape shown in dashed lines to be used in locating the cut position on the leaf shown in Figure 1;

Figure 3 is a top plan view of the leaf shown in Figure 1 with the cutter profiles located in cut positions;

Figure 4 is a block diagram showing schematically certain general features of a system for providing cigar wrappers cut from tobacco leaves;

Figure 5 is a block diagram outlining control concepts employed in the system;

Figure 6 is a flow chart block diagram illustrating interfacing concepts between the computer and the programmable controller as shown in Figure 5;

Figure 7 is a top plan view of an embodiment of the present invention illustrating the various structural features of a system for locating the cuts on a natural tobacco leaf, transferring the tobacco leaf to a cutting platen, cutting the cuts from the natural tobacco leaf and conveying the cuts or wrappers to a successive position for processing;

Figure 8 is a schematic view taken generally along lines 28-28 of Figure 7;

Figure 9 is an enlarged cross-sectional view taken generally along line 29-29 of Figure 7;

Figure 10 is an enlarged plan view of the cutting head taken generally along line 30-30 of Figure 9;

Figure 11 is a side cross-sectional view taken generally along line 31-31 of Figure 9;

Figure 12 is a side elevational view showing the cutting platen in the cutting position;

Figure 13 is an enlarged cross-sectional view taken generally along line 33-33 of Figure 7;

Figure 14 is a graphic view of the cutting table employed in the preferred embodiment of the present invention;

Figure 15 is a cross-sectional view of the cutting table as illustrated in Figure 14;

Figure 16 is a top plan view showing in more detail the cutting platen and cutting table employed in the preferred embodiment of the present invention;

Figures 16A and 16B are plan views illustrating the operating characteristics of the structure shown in Figure 16;

Figure 17 is a cross-sectional view of the digital motors in the structure illustrated in Figure 16;

Figure 18 is a cross-sectional view taken generally along line 38-38 of Figure 17; and

Figure 19 is a schematic logic diagram illustrating operating characteristics of the structure shown in Figure 17.

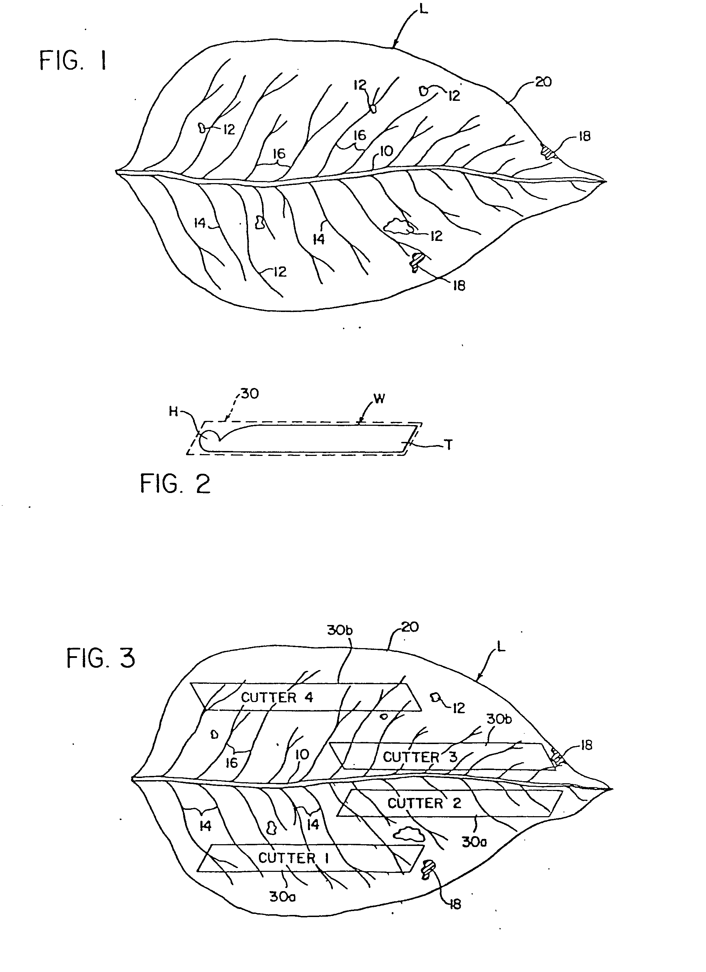

Referring to Figures 1 to 3, a natural tobacco leaf L has known physical characteristics in that it is flaccid, pliable and has internal colour variations, energy ray detectable variations and defects like holes, and dark or black areas. In addition, the natural tobacco leaf has a top surface which is to be used as the exposed portion of the wrapper to be cut from the leaf. Natural leaf L is illustrated as including a centre stem 10 which _____

extends in a somewhat undulating path longitudinally through the leaf, a plurality of holes 12 of varying size, diagonally extending right and left veins 14, 16, respectively, dark areas 18 and an outline or edge 20. The dark areas 18 may be on either surface of the leaf and are visible from the top surface to a degree determined by their location. Indeed, the dark areas may be internal of the leaf and present only a slight color variation when viewed from the top surface of the leaf. Even in that situation, the dark areas could be considered unacceptable for a wrapper of a cigar. Also, abrupt surface color variations can occur as pronounced light areas in the leaf which may be detectable from one or both surfaces of the leaf and which may be undesirable for a cigar wrapper. From the leaf L cigar wrappers W, as shown in FIGURE 2, are to be cut by a cookie cutter type die or clicker die commonly used for cutting of flat sheets in other industries. Wrapper W has a precise shape which allows spiral winding of the wrapper around the binder and/or core of the cigar without producing wrinkles and surface defects. The wrapper generally includes a head H and a portion T called a "tuck". Tuck T is ultimately located in the end of the cigar to be lighted, whereas head H is twisted and wrapped into the portion of the cigar to be held in the smoker's mouth. These shapes are critical and it should be free of dark areas, any holes and heavy veins. The shape of wrapper W, which varies for different length and shaped cigars, can be circumscribed by a four-sided profile 30. The profile is different for left and right hand wrappers and are configurations 30a, 30b, as shown in FIGURE 3. The cutters are labeled CUTTERS 1-4. In this illustrated embodiment the cutters outlined by the shapes 30a, 30b represent cut positions of the same wrapper shapes. In some instances, two or more wrapper shapes could be cut from a single leaf. For the purpose of describing the present system, the cutter to be used in the wrapper has the shape of wrapper W; however, the profile 30 or profiles 30a, 30b are used to locate the cut positions for the wrappers on the surface of leaf L in a manner to avoid any unwanted defects in the resulting wrapper within the confines of general profiles 30a, 30b. In producing wrappers W from leaf L the wrapper profile must be located on the leaf to avoid defects such as holes, the stem, dark areas, color variations of a given magnitude and other such contingencies to produce a wrapper having the quality set forth in the introductory portion of this application. In most arrangements for cutting the wrapper W from a natural tobacco leaf, the stem is removed and the leaf halves are booked or stacked according to the right hand vein arrangement or the left hand vein arrangement. The present system does not contemplate the stemming of the leaf; however, it could be stemmed and a half of a leaf could be processed in the present system. Throughout the rest of the discussion of the present system, wrapper profile 30 is used to locate the cut positions on leaf L of the cutter for a wrapper W since the placement of this profile at various non-conflicting locations will provide proper cut positions for the circumscribed actual wrapper configuration. In practice of the present system, the actual profile 30 or profiles 30a, 30b are slightly greater in size than the contour of the wrapper cutter so that the cut made by the cutter itself will not be at the edge of the leaf or closely adjacent a prohibitive defect to be excluded. In other words, the profile used in selecting the cut positions in accordance with the system herein described is slightly larger than the actual cutter profile used in making the wrapper cut in leaf L so that the cut is offset at least a small amount from the outline of profile 30.

In accordance with the present system, a profile or image of the leaf including the outline, stem, surface variations and defects is created and recorded and stored. Thereafter, the profile 30 is manipulated within stored profile or image to locate positions in which the profile 30 avoids unwanted defects and remains in the confines of the leaf. These selected cut positions are then used to control a cutting machine that positions the leaf and wrapper cutter in the desired relative position for a cutting operation to remove the wrapper from the area bound by profile 30 in its final selected position or positions.

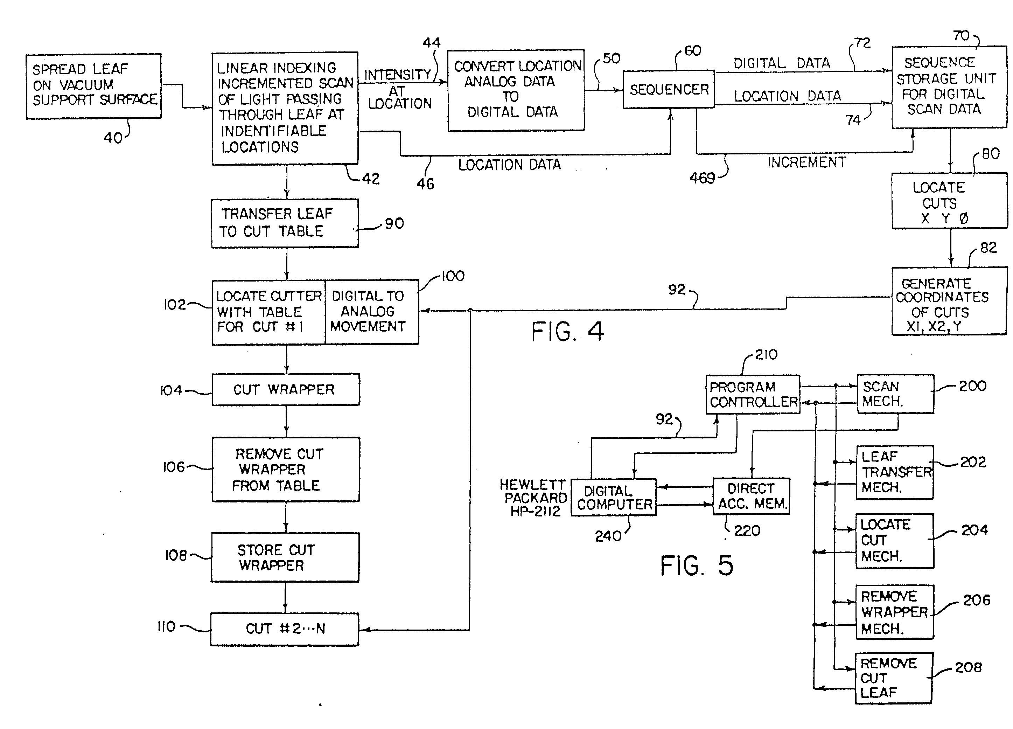

A general functional block diagram of the overall system is schematically illustrated in FIGURE 4. A description of this block diagram will provide general information regarding the basic concept of the system hereinafter described and can be used as an over view of the system of cutting wrappers W from desired positions on leaf L without requiring the previously used manual manipulation and without sacrificing quality of the resulting wrappers. In accordance with the functional block diagram, a natural leaf is spread onto a vacuum support surface which preferably is capable of transmitting light rays so that the rays pass simultaneously through the surface and through the leaf. These rays are read by a light intensity sensitive device which determines the light intensity at selected locations on the support surface. The total light intensity pattern provides a light intensity profile or image of the natural tobacco leaf supported in a spread condition on the surface. The concept of spreading the natural leaf onto a vacuum support surface for cutting is known and is represented by block 40. The concept of passing light through the leaf supported on a vacuum

surface ~~is novel and~~ is represented in block 42. As indicated
by block 42, the leaf is indexed or moved incrementally in one
direction (hereinafter called the Y direction) and the light
intensities at selected areas of the leaf across another
direction (hereinafter called the X direction) are recorded
at identifiable locations identifiable by both X and Y
positions on an imaginary grid on the vacuum surface. The
intensity of the light passing through the leaf at these
various identifiable locations which cover the total leaf
as it has been moved are indicative of the contour of the
leaf and color variation or defects including holes, dark
areas, the stem, the veins and other surface color variations.
By passing light through the leaf, variations on both surfaces
are detected as are variations within the leaf which can cause
discernible color variations that would be objectionable in a
wrapper for a cigar. Block 42 is indicative of this scanning
operation for obtaining light intensity at selected locations
encompassing the total surface area of natural tobacco leaf
L. The scanning operation creates a series of light intensity
signals which are each analog voltages. One voltage signal
corresponds to each scanned location and appears in line 44.
Digital data indicative of the location of the scanned
locations simultaneously appears in line 46. The analog light
intensity signal in line 44 is converted to digital data
indicating whether or not the scanned location has a light
intensity value indicative of white, black or an intermediate
color, termed "gray". Thus, each of the scanned locations
on the leaf, which may be as small as desired and is known as
a Pixel, will produce a light intensity, digital signal in
line 50. The Pixel data will be white, black or gray. If
desired, variations of gray could be obtained; however, such
resolution is not required in the present system. The address data in line 46 identifies the location of the Pixel being detected and transmitted in line 50. As can be seen, if the leaf is segregated into a large number of identifiable locations, or Pixels, which have known addresses (in the X and Y directions) and known light intensity conditions, such as white, black or gray, a profile of the leaf itself can be constructed remote from the leaf. For illustrative purposes, a sequencer 60 is indicated as transmitting the digital information regarding the color of the Pixel and the location of the Pixel to any type of storage unit 70 by lines 72, 74. The particular storage arrangement will then contain a matrix indicative of the profile of the leaf including an outline and defects. This data matrix storage unit 70 is indicated to be a sequenced unit incremented by line 469 for each Pixel in the X direction. In practice, storage unit 70 is a direct accessed memory usable by a digital computer in accordance with known computer practice. The indexing by each X Pixel will cover all Y indexes in sequence. Proceeding further into the schematic representation of the general system as shown in FIGURE 4, cut locations or positions are determined as represented by block 80 using the stored digital data in storage unit 70. This can be done in a variety of computer arrangements which will be described in more detail later. Basically, a digital representation of the cutter profile 30 is compared to the stored digital profile of leaf L in storage unit 70 and these two profiles are shifted until the digitized profile 30 does not include a defect, such as a hole, edge of the leaf, or undue color variations. After one cut position is located, a next profile 30 is also shifted with respect to the digitized leaf profile or image in storage unit 70 to locate still a further cut position which avoids the defects set forth above. This process is continued until the digitized representation of profile 30 is set to the extent possible to obtain the desired number of cuts in leaf L. The various cut locations so located by shifting the digitized profiles 30 within the digitized leaf profile of storage unit 70 are in the form of X, Y and $\emptyset$ corresponding to the X, Y location of a selected point on a line of profile 30 and the angle this line makes with the scanned Y direction. The X, Y and $\emptyset$ coordinates are then converted to usable cut coordinates represented as X1, X2 and Y by a coordinate arithmetic conversion. This conversion is determined by the usable data necessary to obtain the same X, Y and $\emptyset$ location on the cutting machine when a linear converting mechanism is used for purposes to be explained later. Arithmetic conversion to cut coordinates usable in the specific machinery contemplated for actually making the cut in the leaf is represented by function block 82. After the scanning operation schematically illustrated as block 42 has been completed, leaf L is transferred to a cutting table. This process is represented by functional block 90. After the leaf has been transferred to the cut platen, the arithmetically generated coordinates from generator 82 are directed through an interface indicated generally as line 92 to a digital-to-analog movement mechanism 100. This mechanism shifts the cut table carrying the leaf to a known cut position determined by coordinates from interface 92. As will be explained later, the movement by mechanism 100 is translated along three axes corresponding to X1, X2 and Y coordinates. The moving to a cut position is represented by block 102. The transfer of leaf L to the cut table is in an oriented position and the cut table is a vacuum surface which holds leaf L in this oriented position. The coordinates from generator 82 operate on the table as they would on the scanning surface. After the leaf and table have been shifted to the first cut position, the wrapper cutter is shifted downwardly to cut a cigar wrapper from the leaf in this first cut position. This action is represented by functional block 104. The cutter is then raised to remove the wrapper from the leaf, which leaf is still held on the table by vacuum. The wrapper is held by a vacuum means as the cutter moves upwardly. This removes the wrapper from the cutting table as represented by functional block 106. Thereafter, the wrapper is removed from the cutter by a vacuum transfer arrangement in accordance with known wrapper handling procedures. This functional step is illustrated as block 108. Interface 92 then causes a recycling of the cutter table to the second cut position and a second wrapper is cut, removed and stored for subsequent use. This procedure is continued until all the cuts located within leaf L have been made. Thereafter, the next leaf is processed.

While the cutting operation is taking place on one leaf, a second leaf is being spread on the vacuum support surface and scanned and the cut positions are being located and the coordinates X1, X2 and Y are being created. Thus, a tandem arrangement is contemplated wherein the scanning is taking place on one leaf while the cutting operation is taking place on a previous leaf. To facilitate this dual operation, a general control system, as shown schematically in FIGURE 5, is employed in the preferred embodiment. The system includes, as basic machine elements, a scanning mechanism 200, a leaf transfer mechanism 202, a locate and cut mechanism 204, a wrapper removing mechanism 206 and a mechanism 208 for removing the spent leaf after all the cuts have been made from the leaf. All of these mechanisms, which will be explained in more detail later, are controlled in accordance with a standard practice by a commercially available programmable controller 210, such as one using an Intel 8080 microprocessor. The sequence of mechanical operations are conducted in accordance with the program of the programmable controller in accordance with standard practice in machine control operation. The scanning mechanism 200 provides information to a direct access memory 220 which has a two-way communication with a standard digital computer 240 that locates the cut positions, converts them to X1, X2 and Y coordinates and provides the coordinates to the controller 210. A two-way communication with the programmable controller is provided to load the cut positions into the programmable controller when the programmable controller is ready to accept the cut coordinates for processing a scanned leaf L. The control and function of information provided in FIGURES 4 and 5 is illustrative in nature to show the general process being performed in accordance with the novel system and a general control arrangement which allows the various mechanisms to be operated to cut wrappers from leaf L. This use of a controller 210 reduces computer time and software and provides a standard type of machine control for the various mechanisms used to perform the sequence of events set forth generally in FIGURE 4.

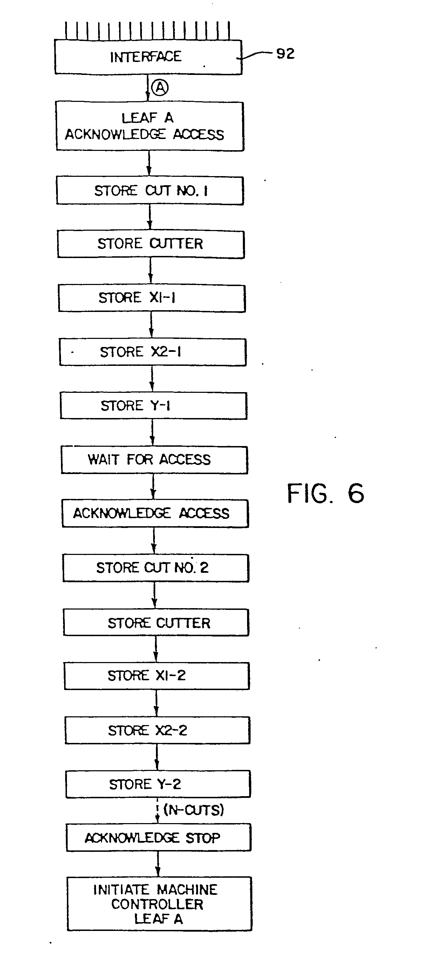

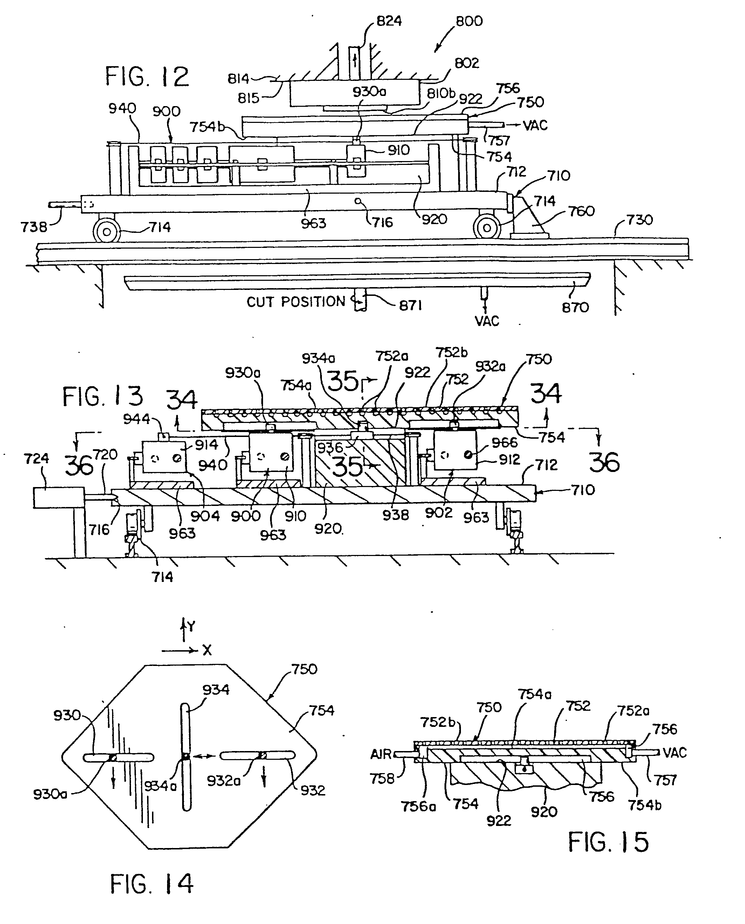

Referring now to FIGURE 6, this figure illustrates, somewhat generally, the interface 92 between the programmable controller 210 and the digital computer 240 as represented in FIGURE 5. Also, the information from computer 240 is illustrated in FIGURE 13. As a first step, the programmable controller 210 acknowledges that it desires access to the digital computer 240 for data regarding the cut coordinates for leaf designated as "leaf A". Thereafter, the programmable controller obtains and stores the number of the cut (1, 2 ... n), the particular cutter (cutter 1, 2, 3, etc), the coordinates X1, X2 and Y for the first cut. It is possible to use different cutters to obtain different wrapper sizes. For that reason, the particular cutter to be used is inputted to the programmable controller. In addition, as noted in Figure 3, there are left and right hand wrapper cutters to accommodate the different vein configurations. Thus, if a cut is to be made from one side of the leaf, one cutter is selected, whereas another cutter is selected for a wrapper from the other side of the leaf. Because of this, the programmable controller receives information regarding which cutter is to be used, which cuts are to be made and where the cut is to be made on the leaf. Thereafter, the programmable controller waits for access again from the computer, acknowledges access and receives the information regarding the second cut. This continues until the programmable controller receives information regarding all n cuts to be made in leaf A. At that time, the programmable controller acknowledges that all information has been received with respect to leaf A and proceeds to initiate the controller for processing leaf A. After leaf A has been processed, the programmable controller then receives information regarding the cut positions and cutters from the digital computer for making the cuts in the next leaf. The cutting operation is proceeding as the scanning operation for the next leaf is proceeding. Figures 4, 5 and 6 are to be taken together to illustrate one arrangement for accomplishing the system contemplated herein. The details of the system are set forth in Application No. 79300323.7 to which reference is directed. However, it should be appreciated that there are several machine control arrangements and computer arrangements which can be employed in practising the method and system contemplated by the description herein.

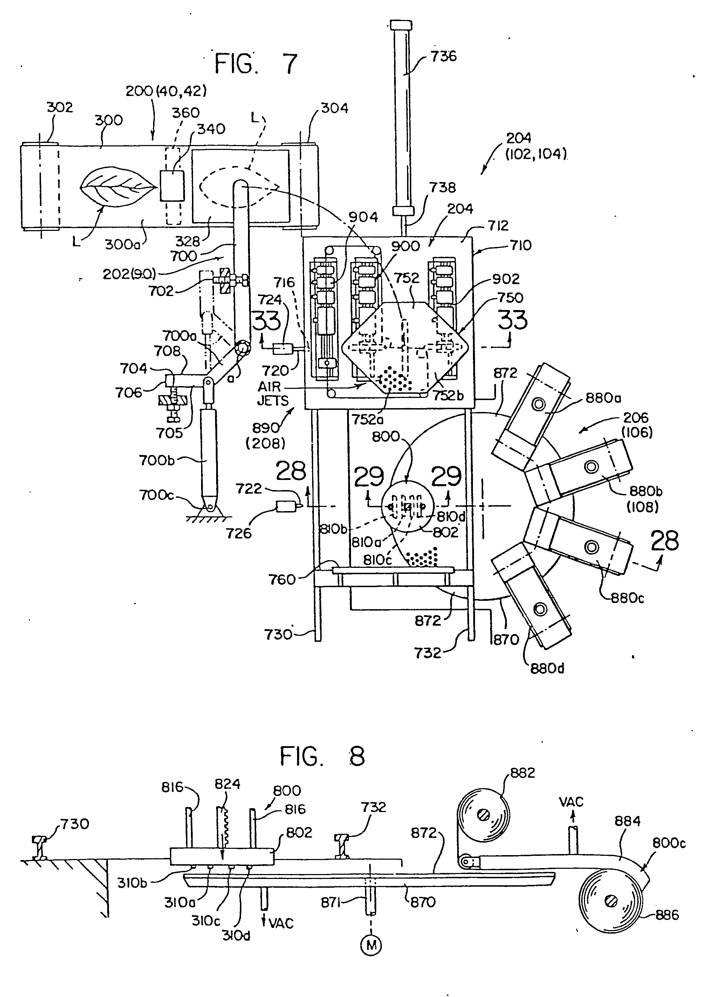

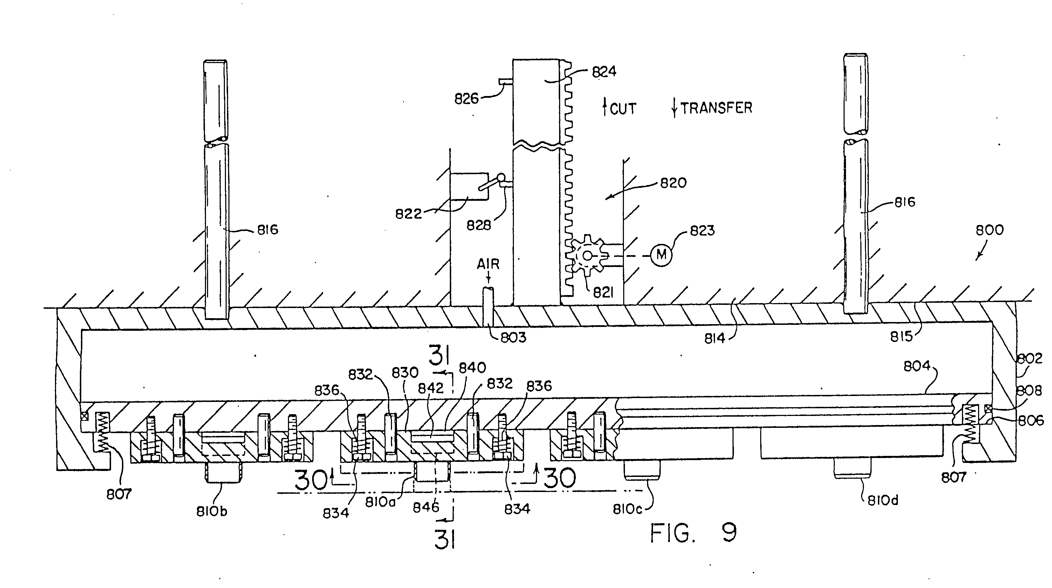

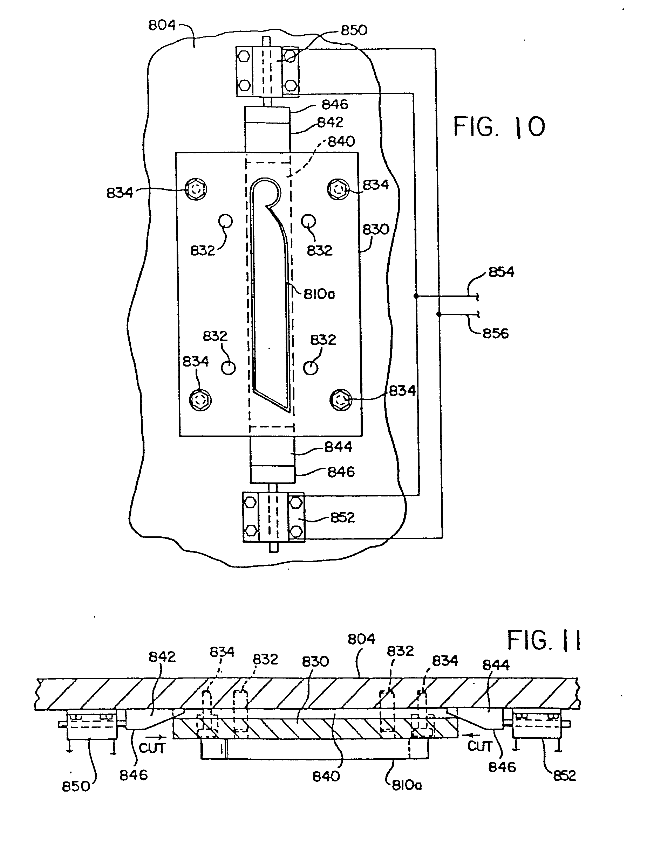

Referring again to Figure 5, the apparatus for performing the scanning and cutting process to remove cigar wrappers from leaf L includes several machine components which are generally labelled in this control concept diagram and are shown in detail in Figures 7-19. Essentially, the programmable controller 210 receives co-ordinates X1, X2 and Y for each of several cuts to be taken from the leaf together with the proper cutter and the sequence of cuts to be made. This information is received in digital form with the three coordinates in the preferred embodiment being binary representations of translation or linear movement. Programmable controller 210 uses the binary coordinates and the digital information to control the various mechanisms including the scanning mechanism previously described to process the leaf so that the computer itself can be used primarily for determining the cut positions based upon direct stored memory information indicative of the profile or image of the leaf after scanning by mechanism 200. The machine cycles controlled by the programmable controller are in accordance with common machine control techniques; therefore, the mechanisms will be described in accordance with their structure and functions.

Scanning mechanism 200 is described in Application No. 79300323.7, therefore, the detail of mechanism 200 is not to be repeated. Details of the other machine components will hereinafter be set forth in an embodiment of the invention to perform the desired functions.

As shown in Figure 7, the leaf is transferred from surface 300a of belt 300 by a somewhat standard vacuum type transferring arrangement which includes an arm 700 supporting a head 328 which includes a lower perforated surface generally parallel to the upwardly facing surface 300a of belt 300 in the transfer area of the scanning arrangement 200. The arm 700, which directs either air or vacuum to head 328, is essentially transverse of the belt 300 when in the transfer position. Head 328 is shifted by arm 700 between a first leaf receiving position and a second leaf releasing position. Arm 700, in the illustrated embodiment, is turned by lever 700a about axis a by a cylinder 700b mounted on fixed trunnion 700c. The first leaf receiving position of arm 700 and head 328 is located by an adjustable stop 704 coacting with surface 705 of extension 706. A second adjustable stop 702 coacts with surface 708 of extension 706 to locate head 328 in the leaf releasing position. Limit switches at these stops will indicate when the transfer head 328 carried by arm 700 is in either of the two positions. Appropriate valving will direct either air or vacuum to the perforated under surface of head 328 for lifting a scanned leaf from belt 300 or depositing a leaf onto the cutting table. The belt must be in a known position with respect to the scanning operation when the leaf is removed from the belt. In this manner, the position of the leaf on the transfer head 328 is correlated and oriented to the Pixels scanned during the scanning operation. This can be done by stopping belt 300 at a fixed number of encoder pulses derived from the motor driving the belt. Other systems could be developed for assuring that the leaf is captured on head 328 in direct correlation to the X and Y scanning lines of the scanning operation. Irrespective of the technique used, leaf L is picked up by transfer head 328 in a fixed position with respect to the prior scanning operation which maintains the positional orientation with respect to the prior scanning of the leaf. Adjustable stop 704 allows small adjustments in the pick up position. Thereafter arm 700 is shifted by cylinder 700b against stop 702 which transfers the leaf held against the lower surface of head 328 to a fixed, known position with respect to platen 710 having an upper surface 712. Small adjustments can be made by stop 704. Leaf L is deposited onto platen 710 in a known position with respect to the prior scanning operation. Platen 710 includes lower guide wheels 714 and a side locator hole 716 into which a pin 720 or 722 is protruded by an operator, such as a solenoid 724, 726, respectively. In the leaf transfer position of platen 710, as shown in FIGURE 27, pin
720 is in hole 716 so that the platen is in a
fixed, known position.  Transversely extending support
rails 730, 732 allow movement of platen 710 by
appropriate means schematically _____

represented as cylinder 736 having a movable rod 738.
Carried upon the upper portion of platen 710 is a cutting table 750 onto which the leaf is transferred when
arm 700 is against stop 702. Cutting table 750 is in
the oriented fixed position as shown in Figure 27 for
receiving the leaf. By coordination of the table 750
and the pick-up position of leaf L from belt 300, the
leaf is deposited by transfer head 328 onto table 750
in a coordinated, oriented position with respect to
its previous scanned position. The table 750 is
supported on platen 710 which is held in the leaf
receiving position by pin 720 entering guide slot 716.
The scanned leaf which produced the desired profile in
a stored memory unit is transferred onto the oriented
table 750 by applying a vacuum to the cutting table
and pressurizing or evacuating the head 328. This is
a known transfer arrangement for tobacco in the cigar
making industry. As best shown in Figures 32-35,
cutting table 750 includes a flat upper nylon member,
or plate, 752 having a plurality of parallel arranged
openings 752a which have a diameter of approximately
0.04 inches (1 mm). This plate, except for holes 752a,
is a cutting board produced by Boston Cutting Die
Company and is formed from hard nylon with a trademark
WOGULON. Upper plate 752 has an upper cutting surface
752b. A second hard nylon plate 754 is formed with
a plurality of generally parallel grooves 754a that
correspond with openings 752a and have a thickness of
about 3/16 of an inch (4.76 mm). Inbetween the
grooves the upper and lower plates 752, 754 are secured together for cutting rigidity. Grooves 754a do
not extend to the periphery of cutting table 750 and
they form a plenum chamber communicated with the openings 752a to allow a vacuum from manifold 756 to be
applied to all grooves 754a and thus to the openings 752a at surface 752b. / Another manifold 756a may be positioned at the opposite end of grooves 754a. In the illustrated embodiment shown in Figure 35 a vacuum is applied to manifold 756 so that a vacuum can be directed to the upper surface 752b of cutting table 750 for clamping a transferred leaf onto the upper cutting surface for performance of the cutting operation. To remove a spent leaf from the surface 752b after the cuts have been made air pressure can be directed to grooves 754a by line 758 communicated with manifold 756a. Of course, a single manifold could be used at one end of the grooves with either vacuum or air being applied selectively to the same manifold. The manifolds are positioned generally outside the cutting area of cutting board or table 750 so that the manifolds do not adversely affect the rigidity of the board or table for the cutting operation. By providing the parallel grooves to direct pressure to the surface 752b of the cutting board or table 750, the cutting table is essentially a rigid, hard nylon cutting structure. As can be seen, vacuum holds the leaf onto the cutting board in the position to which it is transferred by transfer head 328. This position is oriented and coordinated with the scanning operation so that the leaf is in the desired position for adjustment with respect to a cutting mechanism to be hereinafter explained.

CUTTING MECHANISM

Before describing the system for adjusting table 750 to the desired position for cutting, it is desirable to understand the preferred embodiment of the cutting mechanism 800. This mechanism is best shown in Figures 29-31 and includes a pancake cylinder 802 having a pneumatic inlet 803 and a piston 804 movable against a lower abutment or shoulder 806 and spring biased away from the abutment by a plurality of circumferentially spaced machine springs 807. Of course, an appropriate seal 808 is employed to seal the cylinder and piston. As illustrated, four separate cookie cutter type dies 810a-d are provided on piston 804. In practice, it is conceivable that only a left hand and right hand cutting die will be used. By showing four separate cutting dies, the system can cut two separate sized wrappers from each half of the leaf by an appropriate signal from the computer to the controller. This is useful when a large wrapper is being cut from the natural tobacco leaf and it is found that a smaller wrapper could be cut from available non-defected areas instead of one or more larger wrappers. The use of more cutting dies would increase the productivity; however, it is not necessary for the general understanding of the system. Basically, the computer commands the programmable controller 210 where to cut and which cutter 810a-d to use. Normally, the cutters 810a and 810c would be used to produce wrappers having the same general shape, but cut from opposite sides of the leaf so that they would be opposite profiled wrappers for use in different cigar making machines. The cutters themselves are fixed in position and the table 750 adjusts the tobacco leaf carried thereon to the proper cutting position. In the illustrated system, cylinder 802 is mounted on a fixed machine frame 814 having a lower abutment surface 815. A plurality of slidable guide pins 816 allow reciprocal movement of cylinder 802. A mechanism 820 is provided for shifting cylinder 802 between the upper cutting position with the cylinder against surface 815 and a lower position for transferring the cut wrapper to a subsequent apparatus for storage and use. Mechanism 820 could take a variety of forms; however, in the illustrated embodiment this mechanism includes a limit switch 822 to control the vertical position of cylinder 802.    A pinion 821
driven by an appropriate motor 823 coacts with rack 824
secured on cylinder 802 to drive the cylinder between
the upper cutting position and lower transfer position.
The basic location of these two vertical positions is
generally controlled by an appropriate mechanism, such
as cams 826, 828 coacting with limit switch 822.
During the cutting operation, cylinder 802 is abutting
against surface 815 to rigidify the cylinder.    During
the wrapper transfer operation, rack 824 shifts cylinder 802 downwardly.

        Cutters 810a-810d are located on piston 804 and
are essentially the same in structure, except the size
and/or shape of the cutters are somewhat different to
create wrappers having the desired shape.    It is appreciated that any . number of cutters could be mounted on
the piston and can be selectable for the actual cutting
operation.    Since each of the cutters, except for
shape, is the same, only cutter 810a will be described
in detail.    This description will apply equally to the
other cutters mounted on the piston.    Indeed, only one
cutter may be provided in some instances.    Cutter 810a
is fixed to a cutter block or support block 830 slidably received upon a plurality of dowl pins 832.
Appropriately spaced machine bolts 834 include machine
springs 836 which hold block 830 rigidly against the
undersurface of piston 804.    A slot 840 extends across
the back surface of block 830 in a position generally
parallel to the wrapper cutter 810a.    Opposed cams 842,
844 having a cut supporting upper surface 846 are movable by solenoids 850, 852 under the control of a signal
received by lines 854, 856.    If a particular cutter is
selected to make the cut being processed by the mechanism as shown in Figure 27, solenoids 850, 852 shift
cams 842, 844 inwardly until surfaces 846 project block 830 outwardly on dowl pins 832. In this fashion,
surfaces 846 support cutter 810a in a downwardly
extended active position with respect to all other
cutters which remain in a retracted inactive position.
Thus, only cutter 810a will cut when piston 804 is
forced downwardly by pressure from inlet 803 against
the action of machine springs 807. The particular
signal indicating the cutter to be used controls the
signal within lines 854, 856 to select any one of the
cutters supported on piston 804. When piston 804 is
forced downwardly against table 750, the extended cutter
cuts according to the adjusted position of the table
with respect to that activated cutter. As the piston
804 is retracted by exhausting air from line 803, the
cigar wrapper cut by cutter 810a is held within the
cutter and pulled from the surface of leaf L. To
assist in this action, a vacuum, from a source not
shown, may be directed to the inside or the interior
of the cutter. With the wrapper cut and within the
cutter, cylinder 802 can be shifted downwardly after
platen 710 is retracted from the cutting position. The
wrapper is now ready to be transferred for subsequent
processing.

TRANSFER OF CUT WRAPPER

Referring now to FIGURES 7 8 and 12 a
cut wrapper is transferred to the vacuum table 870
indexable about center shaft 871. The upper surface
872 of the table is perforated at least in selected
areas. A plenum chamber below surface 872 can apply
a vacuum to the surface in accordance with standard
practice. Of course, certain areas of the plenum could
be formed to create surface vacuum, air pressure or
atmospheric pressure to assist in wrapper transfer.
When cylinder 802 is moved downwardly by rack 824,
cutter 810a having a cut wrapper therein is positioned
onto surface 872. Thereafter, air pressure can be
applied to the cutter profile or the vacuum itself in the table 870 can draw the cut wrapper from the cutter onto surface 872. Cylinder 802 is then retracted upwardly for the next cutting cycle and table 870 is rotated or indexed to one of the standard wrapper storage bobbins 880a-880d. Each of the bobbins receives a cut wrapper from only one of the cutters 810a-810d. The standard wrapper storage bobbins 880a-880d each includes a porous belt 882 on a storage reel, a vacuum transfer and holding housing 884 and a storage bobbin 886, as shown in FIGURE 8. After a wrapper cut has been made, the wrapper is deposited onto table 870 which is indexed to the desired position where a vacuum is applied to belt 882 at least adjacent the inlet end of housing 884. The cut wrapper is transferred to belt 882 and held onto the belt until it is reeled onto bobbin 886. During the indexing of table 870 and storage of a cut wrapper the next cutting operation is being performed by mechanisms 204.

This wrapper removal procedure follows each cut and is repeated until all cuts are made in leaf L. The wrappers are all stored on the appropriate bobbins 880a-880d. Platen 710 carries table or cutting board 750 back to the leaf receiving position shown in Figure 7 where the table 750 is pressurized by line 758 and air jets 890 blow the cut leaf off the table into an appropriate repository. This removing mechanism using jets 890 is schematically illustrated as block 208 in FIGURE 5.

## CUT LOCATING MECHANISM

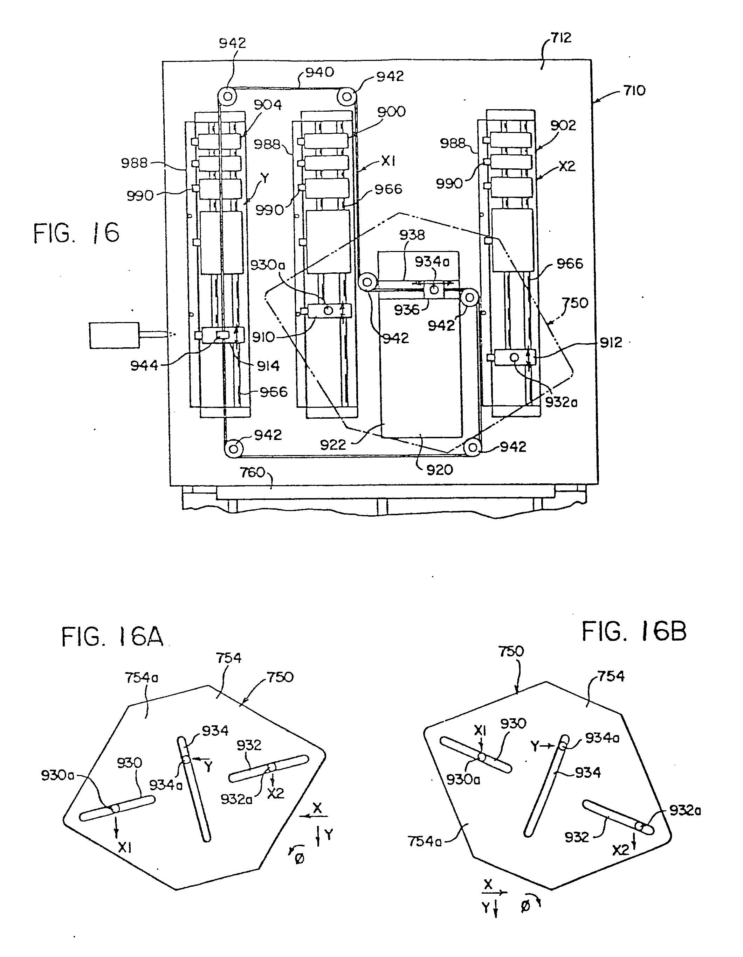

After the cut positions have been located and identified as three digital words (X1, X2, Y), these words or co-ordinates are used to locate the cut positions of cutting board or table 750. To perform the movement of table 750 to the various cut positions platen 710 carries table 750 to the position shown in FIGURE 32. During movement of the platen
to the cutting position, or after the movement has
been completed, table 750 is shifted, rotated and
otherwise moved to align leaf L directly below the
selected cutter, which for illustrative purposes has
been indicated to be wrapper cutter 810a, in the cut
position identified by coordinates X1, X2 and Y. This
shifting of table 750 is done by converting the three
digital words (X1, X2, Y) corresponding to the
proper cut position into three coplanar translation
movements of the table 750 which duplicate the X, Y
and $\emptyset$ cut position previously described. In
accordance with a novel aspect of the present system,
the three linear movements, which could be
coordinates X, Y and $\emptyset$ as shown in FIGURES 16A, 16B
are obtained by linear translation distances
determined by the binary number of the three separate
words X1, X2 and Y. Of course, according to the
type of moving mechanism used for table 750, the
translation magnitude words will vary. The
translation distances cause table 750 to move so that
the located X, Y, $\emptyset$ position of leaf L is aligned
with the selected cutter. In accordance with this
aspect of the system, no rotary movement is required
for table 750. The angular movement corresponding to
$\emptyset$ is obtained by differential linear translation
movements of two linear motors 900 and 902 fixed on
surface 712 of platen 710. The details of these
motors will be explained in connection with FIGURES
17-19. The motors 900, 902 and 904 each carry a
movable element 910, 912, 914, respectively, which are
translated a distance controlled by the binary
magnitude of digital words X1, X2, and Y, respectively.
These words are directly correlated with the movement
of elements 910-914 to locate table 750 in the cut position as determined by the scanning and selecting process previously described. The location of table 750, in turn orients leaf L carried in a known relation on surface 752b. Of course, various systems could be used for movement of table 750, some of which would include a rotary action. It has been found that by using the translation action developed in accordance with the present system, the difficulties encountered in rotating table 750 through an angle determined by a cut coordinate is avoided. As previously discussed, the cut position can be at an angle with Y axis since the stem of the leaf may be at an angle or the leaf may be positioned on belt 300 at a slight angle. For that reason, the preferred system requires a certain amount of angular movement which is obtained by differential translation of the two sides of table 750 by motors 900, 902.

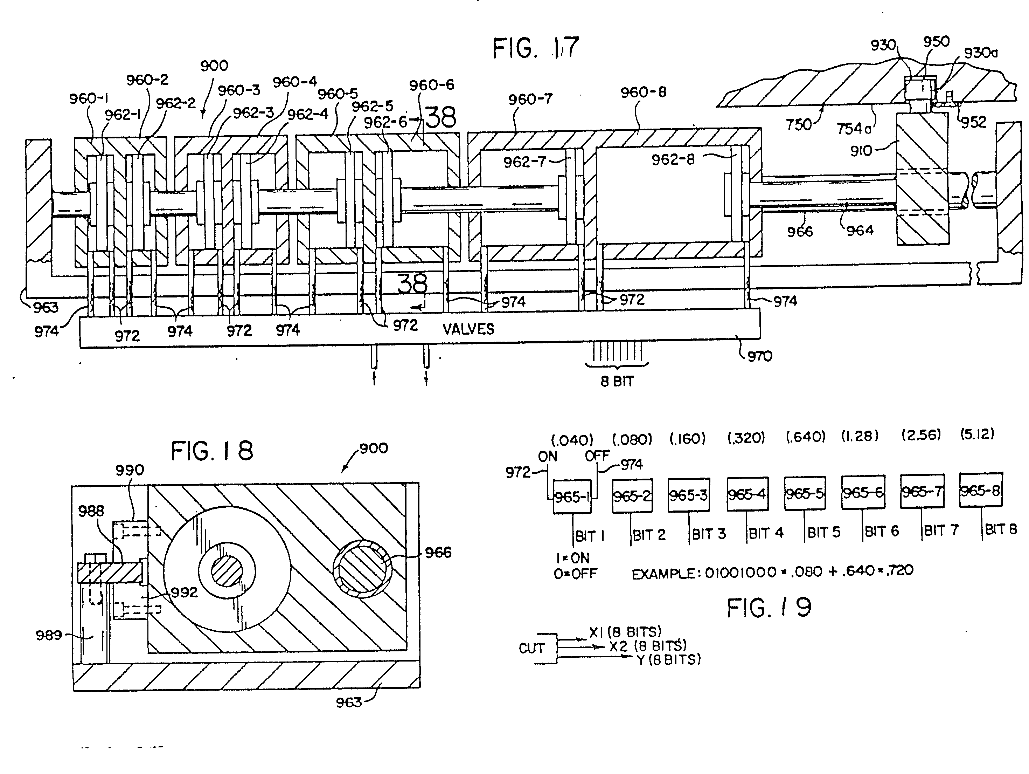

The lower surface 754b of the lower nylon plate 754 forming table 750 rides along and is supported by an anvil 920 having an upper table support surface 922 which is parallel to lower surface 754a. Anvil 920 supports table 750 during the shifting action to locate the various cut positions. To move table 750 into the cutting positions there are provided two generally parallel slots 930,932, as best shown in FIGURES 14, 16A and 16B. These slots are milled or otherwise provided in the lower nylon plate 754 of table 750 and are generally parallel in relationship and extend in the X direction of table 750. Between these two slots there is provided a generally orthogonal slot 934 which is at an oblique angle with slots 930, 932 which angle, in practice, is 90° so that it is aligned with the Y axis of table 750. The
X and Y axes of the table 750 define a grid
corresponding to the scanned grid of the leaf.  Of
course, the grids are not marked on the scan surface
or table since they are correlated by the transfer
of leaf L.  Pins 930a, 932a, and 934a are slidably
received within slots 930, 932, and 934, respectively
so that movement of the pins in a direction generally
transverse to the slots will orient table 750 for
proper positioning of leaf L at the proper cutting
position with respect to the selected cutter,
illustrated as cutter 810a.  Pins 930a, 932a move in
straight lines on platen 710 which lines are parallel
to the Y direction of table 750 when the table is in
the leaf receiving position shown in FIGURES 17 and 14.
Pin 934a is movable in a straight line on platen
710, which straight line is parallel to the X
direction of table 750 when the table is in the
leaf receiving position shown in FIGURES 17 and 14.
In the illustrated embodiment, as best shown in
FIGURE 14, slots 930, 932 are aligned and on opposite
sides of slot 934 and pins 930a, 932a and 934a are
all aligned in a direction corresponding to the
X direction of table 750 when the table 750 is in its
leaf receiving position shown in FIGURES 7 and 14.
Pins 930a, 932a are directly supported upon the
movable elements 910, 912, respectively, of motors
900, 902, respectively.  Consequently, reciprocation
of elements 910, 912 causes reciprocation of pins
930a, 932a to reciprocate table 750 in the directions
indicated by the arrows in FIGURE 14.  Pin 934a is
carried by a block 936 slidably received within
slot 938 of anvil 920.  A drive cable 940 with a
series of pulleys 942 connect block 936 drivingly with an anchor block 944 supported on element 914 which is reciprocated by the third translating motor 904. Slot 938 is orthogonal to the rest position of slot 934 and parallel to the direction of slots 930, 932. Thus, translation of element 914 causes pin 934a to move in the direction indicated in FIGURE 14 a distance equal to the translation distance of element 914.

As three digital words are received, they indicate in binary language the amount of translation of the elements 910, 912, 914. The words actually provide the translated position of elements 910-914 for the cut position. If the motors 900-904 start from a zero position, the actual word gives the amount of translation. If the elements are in an intermediate position the words indicate the corrective amount of translation. Motors 900-904 may be at a prior cut position and they need not retract to the zero position to go to the next cut position. In practice, table 750 is in the zero positions of X1, X2 and the middle position of Y when in the cut receiving position shown in FIGURES 7 and 14. The binary words X1, X2 and Y are converted into movement of table 750 by the coaction between slots 930-934 and pins 930a-934a. In FIGURE 14, table 750 is in the rest or leaf receiving position, which is the position used in accepting a leaf when platen 710 is in the leaf receiving position shown in FIGURE 27. After the leaf has been received and vacuum has been applied through line 757 of table 750, the leaf is held in place and the programmable controller receives cut coordinates X1, X2 and Y which control the translation position of elements 910-914 controlled by motors 900-904, respectively. In this manner the table 750 carrying the leaf is adjusted with respect to the cutter 810a, which cutter is then forced downwardly against table 750. When this happens, the upper surface 922 of anvil 920 supports the table to create a reactive force against the table to facilitate the cutting action. Thus, the movement of the cutting board or table 750 into the proper cut position is by the movement of three pins, which are translated, but do not support the table in a vertical direction. The actual cutting force is against anvil 920 with upper portion 752 of table 750 being the actual cutting member. In this manner, table 750 has a low weight and develops low inertia forces. Pins 930a-934a need not have a heavy construction to withstand the subsequent cutting forces. Thus, the pins develop low inertia forces. In practice, a head is provided on the moving pins 930a,932a to coact with a retaining structure adjacent slots 930,932 to hold table 750 in the horizontal position as it is shifted. As previously mentioned, after the cut has been made the wrapper is removed by the cutter and a platen 710 moves to a position clearing cylinder 802 so that the cylinder can be moved downwardly to transfer the cut wrapper onto vacuum table 870 for subsequent indexing to one of the wrapper bobbins 880a-880d. When the cut is made by cutter 810a, table 870 is indexed to wrapper storage mechanism 880a where the cut wrapper is transferred to storage element or bobbin 886 for subsequent loading into a cigar machine in accordance with standard cigar making practice. As shown in FIGURE 17, the top of the pins 930a, 932a can include a head 950 held within the respective slots by an appropriate plate 952. Other arrangements, such as a T-slot and head, could be used to hold table 750 onto the moving pins for movement therewith. The actual cutting action takes place against the upper surface 922 of anvil 920.

FIGURE 16A shows an example where table 750 has been shifted to a cut position with X1 greater than X2 and Y shifted to the left, as viewed from surface 754a. Another example is shown in FIGURE 36B with X2 greater than X1 and Y shifted to the right as viewed from the under surface 754a.

A variety of structures could be used to translate elements 910-914 in a manner controlled by digital signals; however, one mechanism for accomplishing the conversion between digital co-ordinates in binary words and translation of the movement elements is a binary motor concept. This concept is used in motors 900-904. Since each of these motors is essentially the same, only motor 900 will be described in detail and this description will apply equally to binary motors 902 and 904. As shown in FIGURES 17-18, motor 900 includes a series of cylinders 960-1 to 960-8 having internal double acting pistons 962-1 to 962-8. These cylinders are connected together so that an eight bit binary word used to control the pressure in each of the cylinders will translate element 910 a distance corresponding to the numerical value of the binary number. Of course, the binary number could include various number of bits. A variety of structures could be used for interconnecting the pistons and cylinders; however, in the illustrated embodiment the cylinders are connected together in the following sets: 960-1 and 960-2, 960-3 and 960-4, 960-5 and 960-6 and 960-7 and 960-8. Piston 962-1 is fixed at one end of motor frame 963. The pistons are interconnected in the following sets: 962-2 and 962-3, 962-4 and 962-5; 962-6 and 962-7. Piston 962-8 is connected to the movable element 910 by shaft 964. Successive pistons have strokes which vary in binary fashion, i.e. vary as a factor of 2. In the illustrated embodiment as set forth graphically in FIGURE 19, the first cylinder has a stroke of 0.04 inches (1mm). Each of the successive cylinders has strokes which are factors of 2 of this stroke. A support and bearing rod 966 extends the complete length of motor frame 963 and passes through the respective cylinders as shown in cross-section in FIGURE 18. This rod allows translation of the various cylinders in a direction parallel to the stroke of the pistons within the cylinders. To support the other side of the cylinders, there is provided a fixed rail 988 supported on a fixed stand 989. The cylinder housing includes upper and lower guide plates 990, 992 which slide along the fixed rail 988 to support the cylinder housings in the vertical direction. Rod 966 controls the horizontal movement of the motor. To move the pistons in the respective cylinders, there is provided a double acting valving unit 970 including valves 965-1 to 965-8 schematically shown in FIGURE 19. These valves are controlled by the binary logic on bit No. 1 to bit No. 8 of the binary word indicating the amount of translation for element 910. Flexible conduits 972 are the ON or the YES fluid conduits, whereas the flexible conduits or couplings 974 are the OFF or NO conduits. A selected binary word indicating the movement of element 910 controls the valves shown in FIGURE 19 to control fluid pressure to the various cylinders shown in FIGURE 17. By the illustrated example, the amount of movement of element 910 corresponds to the magnitude of the binary number in the 8 bit word directed to the valving unit 970.
Element 910 is translated in accordance with the
binary word used as one coordinate (XI) in locating
the cut position of table 750 with respect to
cutter 810a.   The operation of the three motors
900-904 (X1, X2 and Y) duplicates the located cut
position provided as three binary words (8 bits).
The three motors are moved in accordance with the
relationship of the pins and grooves needed to
locate the upper surface of table 750 for cutting.
Primarily, the cut locating mechanism involves three
translating devices which are translated in
accordance with the desired ultimate position of
leaf L, as indicated by binary numbers or digital
information.

CLAIMS:

1.      An apparatus for cutting an element (W) having a preselected shape from a natural tobacco leaf (L), said apparatus comprising: vacuum means for supporting said leaf in a spread condition and in a random position on a leaf receiving member (300); means (80) for locating at least one cut position on said leaf and oriented with respect to said leaf receiving member; a cutting table (750) including a leaf support surface (752b) and means (754a) for creating a vacuum at said leaf support surface; means (720) for positioning said table in a known leaf receiving position with respect to said leaf receiving member (300); transfer means (202) for transferring said leaf from said leaf receiving member to said leaf support surface when said cutting table is in said leaf receiving position; a cutter (810a) for cutting said preselected shape; means (736) for shifting said table from said leaf receiving position to a leaf cutting position whereat said support surface of said table (750) is spaced from said cutter; means (102) for orienting said table relative to said cutter (810a) to a position with said cutter aligned with said selected cut position; means (802, 804) for forcing said cutter against said table whereby an element (W) is cut and separated from said leaf; and means (870) for then transferring said cut and separated element to a selected location for subsequent use.

2.      An apparatus as claimed in claim 1, characterised in that said cutter forcing means comprises a piston (804) in a cylinder (802) and means (803) for directing fluid to said cylinder.

- 2 -

3.          An apparatus as claimed in claim 2,
characterised by means (842) for shifting said cutter
with respect to said piston (804) between a retracted
non-cutting position and an extended cutting position.

4.          An apparatus as claimed in claim 3,
characterised in that said shifting means comprises a
cam (842) coacting between said cutter (810a) and
said piston (804) and means (850) for shifting said cam
between a first position with said cutter adjacent
said piston and a second position with said cutter
extended from said piston.

5.          An apparatus as claimed in claim 1 or 2,
characterised in that said cutter forcing means is
arranged to force a member (804) carrying a plurality of
said cutters (810a-d), each with a cutting shape
corresponding to one of a plurality of preselected
shapes, toward said leaf support surface (754b) and
respective means (842, 850) for selectively shifting
each of said cutters with respect to said member (804)
between a retracted non-cutting position and an extended
cutting position.

6.          An apparatus as claimed in claim 5 when
appended to claim 2, characterised in that said member
carrying said cutters comprises said piston (804) of
said forcing means.

7.          An apparatus as claimed in claim 5 or 6,
characterised in that each of said shifting means
comprises a cam (842) coacting with one of said cutters
(810a) and said member (804) carrying said cutters and
means (850) for shifting said cam between a first
position with said cutter adjacent said member (804) and a second position with said cutter extended from
said member (804).

8.        An apparatus as claimed in any one of the
preceding claims, characterised by an anvil (920)
generally aligned with said cutter or cutters for
supporting the table (750) as a said cutter is forced
against said table (750).

FIG. 1

FIG. 2

FIG. 3

SPREAD LEAF ON VACUUM SUPPORT SURFACE — 40

LINEAR INDEXING INCREMENTED SCAN OF LIGHT PASSING THROUGH LEAF AT INDENTIFIABLE LOCATIONS — 42

INTENSITY AT LOCATION — 44

CONVERT LOCATION ANALOG DATA TO DIGITAL DATA — 50

SEQUENCER — 60

DIGITAL DATA — 72

LOCATION DATA — 74

SEQUENCE STORAGE UNIT FOR DIGITAL SCAN DATA — 70

LOCATION DATA — 46

INCREMENT — 469

LOCATE CUTS X Y Ø — 80

TRANSFER LEAF TO CUT TABLE — 90

GENERATE COORDINATES OF CUTS X1, X2, Y — 82

LOCATE CUTTER WITH TABLE FOR CUT #1 — 102 | DIGITAL TO ANALOG MOVEMENT — 100

92

FIG. 4

CUT WRAPPER — 104

REMOVE CUT WRAPPER FROM TABLE — 106

STORE CUT WRAPPER — 108

CUT #2···N — 110

PROGRAM CONTROLLER — 210

SCAN MECH. — 200

92

HEWLETT PACKARD HP-2112 | DIGITAL COMPUTER — 240

DIRECT ACC. MEM. — 220

LEAF TRANSFER MECH. — 202

LOCATE CUT MECH. — 204

REMOVE WRAPPER MECH. — 206

REMOVE CUT LEAF — 208

FIG. 5

INTERFACE — 92

Ⓐ

LEAF A
ACKNOWLEDGE ACCESS

STORE CUT NO. 1

STORE CUTTER

STORE X1-1

STORE X2-1

STORE Y-1

WAIT FOR ACCESS

FIG. 6

ACKNOWLEDGE ACCESS

STORE CUT NO. 2

STORE CUTTER

STORE X1-2

STORE X2-2

STORE Y-2

(N-CUTS)

ACKNOWLEDGE STOP

INITIATE MACHINE
CONTROLLER
LEAF A

FIG. 7

FIG. 8

FIG. 9

FIG. 10

FIG. 11

FIG. 12

FIG. 13

FIG. 14

FIG. 15

CUT POSITION

VAC

AIR

VAC

FIG. 16

FIG. 16A

FIG. 16B

FIG. 17

FIG. 18

FIG. 19

European Patent Office

EUROPEAN SEARCH REPORT

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | DE - A - 2 538 964 (HAUNI-WERKE KÖRBER) <br> * Figures 1,2; pages 6-11 * <br> & US - A - 4 094 325 | 1 | A 24 C 1/04 <br> B 26 D 5/34 |
| P | FR - A - 2 373 240 (ARENCO) <br> * Figures; pages 2-3 * | 1 | |
| P | NL - A - 78 08627 (DOUWE EGBERTS) <br> * Page 2, line 9 - page 3, line 11; page 8, line 4 - page 10, line 14, figures 2a, 2b, 3 * <br> & FR - A - 2 400 852 <br> & GB - A - 2 004 643 | 1 | TECHNICAL FIELDS SEARCHED (Int. Cl.³) <br><br> A 24 C <br> G 06 F <br> B 07 C <br> B 26 D <br> C 03 B |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 09-03-1981 | RIEGEL |

EPO Form 1503.1  06.78